# EUROPEAN PATENT SPECIFICATION

(11) **EP 0 473 790 B1**
(45) Date of publication and mention of the grant of the patent: **15.05.1996**
(21) Application number: 91904799.3
(22) Date of filing: 28.02.1991
(51) Int. Cl.: A61B 17/22, A61B 17/36

(54) **WIRE FOR OPENING OBSTRUCTED PART OF BLOOD VESSEL**
DRAHT ZUM ÖFFNEN EINES BLOCKIERTEN TEILS EINES BLUTGEFÄSSES
FIL DESTINE A OUVRIR UNE PARTIE OBSTRUEE DE VAISSEAU SANGUIN

(30) Priority: 28.02.1990 JP 48657/90
(43) Date of publication of application: 11.03.1992
(73) Proprietor: IMAGE MAKER LTD., Shiga-ken 520 (JP); HACHIDA, Mitsuhiro, Shinjuku-ku, Tokyo 162 (JP)
(72) Inventor: HACHIDA, Mitsuhiro, The Heart Institute of Japan, Shinjuku-ku, Tokyo 162 (JP)
(74) Representative: Türk, Gille, Hrabal, Leifert
(86) International application number: JP9100272
(87) International publication number: WO9112770

(56) References cited:
- EP-A- 0 218 809
- EP-A- 0 219 216
- EP-A- 0 314 896
- WO-A-89/11311
- CA-A- 1 124 155
- JP-A- 6 075 064
- JP-A-52 141 092
- JP-A-61 113 438
- JP-A-62 109 560
- JP-A-62 231 650
- JP-A-62 231 675
- JP-U-62 157 548
- US-A- 2 756 752
- US-A- 3 635 223
- US-A- 4 030 503

## Description

The invention relates to a device for opening (or enlarging) a blood vessel, particularly, an obstructive or stenosed region in a coronary artery, to pass blood flow (or pass much blood flow).

Stenosis of coronary arteries due to arteriosclerosis or the like causes myocardial infarction within a short period of time after having been broken out if the stenosis is left alone. Accordingly, urgent or emergent medical treatment is required.

As a method of medical-treating (opening) the stenosed region, there have conventionally been known a method using a balloon catheter, and a method using a thrombolytic agent or drug.

The balloon catheter is provided with an inflatable balloon at the distal end of a catheter body (refer to JP-U1-61-130240 and JP-U1-61-171941). The balloon catheter is inserted into a vascular stenosed region under the condition that the balloon is constricted, and then fluid such as physiological saline, carbonic acid gas, a contrast medium or the like is injected into the balloon through a passage in the catheter body to inflate the balloon. Thus, the stenosed region is enlarged, therby opening the stenosed region.

As thrombolytic drug, there are known Streptokinase, Urokinase or the like. The thrombolytic drug is directly administrated into the coronary artery, dissolves a thrombus within the artery, and opens the same.

However, the method using the balloon catheter only enlarges tissues of the artery, but does not remove the stenosed region. Accordingly, the balloon catheter method has the disadvantage that restenosis occurs in a relatively short period of time and with a high probability (for example, with a probability of 44% within three (3) months). Further, the method has also the disadvantage that a complication such as a myocardial infarction, cardiogenic shock or injury to the artery or the like occurs.

The method using the thrombolytic drug has a tendency of hemorrhage, gastrointestinal bleeding, intracerebral bleeding or the like as a side effect, and has also a low percentage of success such as about 50%.

JP-A-62 231 675 defines a device for coronary revascularization comprising a catheter and a wire within the catheter according to the preamble of claims 1 and 2.

For guiding a catheter to be introduced into a blood vessel it is known (JP-A-62 231 675) to provide a flexible wire inserted in the catheter to guide the catheter within the blood vessel. The flexible wire is provided at its surface with concaves in order to decrease the contact area between the flexible wire and the inner surface of the catheter so that the wire can be easily inserted into the catheter and thus pain to the patient is relieved. The flexible wire is made of X-ray non-transmittable material so that it can be easily located by X-rays.

For performing enderterectomy an instrument is known (CA-A-1 124 155) having a reaming head at the distal end of a resiliant wire. The reaming head includes a straight rod-like shaft which is arounded at its forward end, an integral helical thread encircling a portion of the shaft of the reaming head.

In view of the above, it is object of the present invention to provide a device for opening a stenosed region of coronary artery, which avoids the defects or the disadvantages described above.

To this end, the invention provides a device having the features of claim 1 or claim 2, respectively. Improvements of this device are subject matter of the respective dependent subclaims.

In particular, the aforesaid object is achieved by the arrangement that a plurality of projections or grooves are formed within a range of the distal end of a flexible or elastic wire body, or the arrangement that a spiral body is provided in spaced relation to the distal end of a flexible or elastic wire body, a portion of the wire body within a range of the spiral body being resected.

The distal end of the wire body, the projections or the spiral body may be made of a high-electricity resistive material or a high magnetic material.

Further, an elastic core may be embedded in the wire body.

Furthermore, the wire body may be bent at a location between the distal end of the wire body and the projections, the grooves or the spiral body.

Moreover, the arrangement may be such that a passage is formed in the wire body, and the passage is open to a surface of the wire body within a range of the distal end of the wire body so that a drug is supplied into the passage. Further, the arrangement may also be such that a glass fiber is inserted through the passage, and a laser is supplied through the wire from the outside.

In the case of the wire for opening the stenosed region of coronary artery, according to the invention, the configuration or shape of each of the projections at a formed section is, for example, a spherical shape, a circular cylinder shape, a spiral shape, a circular truncated cone shape, or the like, and the configuration of each of the grooves is, for example, a ring-like shape, a spiral shape, a semicircular shape or the like. Further, the number of the projections and the grooves at the formed section is optional.

The wire body is made of, for example, a plastic material, and the projections are made of a material identical with that of the wire body, or are made of another material such as, for example, a plastic material different from the first-mentioned plastic material, or are made of a high-electricity resistive material or a high magnetic material, The core consists of a metal wire, for example, which functions to supply high-frequency current, functions to reinforce the wire body, or functions to raise elasticity of the wire body.

In connection with the above, in the wire for opening the stenosed region of the artery, according to the invention, the term "opening the stenosed region of the artery" includes enlargement of a stricture section in a blood vessel.

Embodiments of the invention will be described below with reference to the drawings, wherein for reasons of clarity the catheter is not shown in figures 1-7 and 9, wherein
Fig. 1 is a cross-sectional view of a region of the distal end of a wire for opening a stenosed region of a coronary artery, according to a first embodiment of the invention;
Fig. 2 is a cross-sectional view of a region of the distal end of a wire for opening a stenosed region of a coronary artery, according to a second embodiment of the invention;
Fig. 3 is a cross-sectional view of a region of the distal end of a wire for opening a stenosed region of a coronary artery, according to a third embodiment of the invention;
Fig. 4 is a cross-sectional view of a region of the distal end of a wire for opening a stenosed region of a coronary artery, according to a fourth embodiment of the invention;
Fig. 5 is a cross-sectional view of a region of the distal end of a wire for opening a stenosed region of a coronary artery, according to a fifth embodiment of the invention;
Fig. 6 is a cross-sectional view of a region of the distal end of a wire for opening a stenosed region of a coronary artery, according to a sixth embodiment of the invention;
Fig. 7 is a cross-sectional view of a region of the distal end of a wire for opening a stenosed region of a coronary artery, according to a seventh embodiment of the invention;
Fig. 8 is a view showing a use of the wire of one of Figs 1 to 7 for opening the stenosed region of a coronary artery: and
Fig. 9 is a fragmentary enlarged view of Fig. 8, showing the state that the stenosed region of the coronary artery is opened.

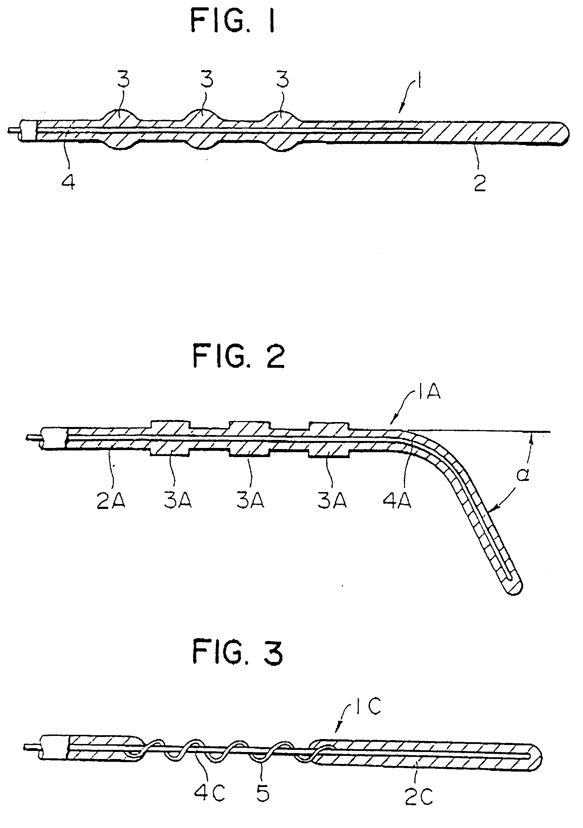

Fig. 1 shows the distal end of an elongated wire 1 for opening an obstructive or stenosed region of a coronary artery, a wire body 2, projections 3 formed on the wire body 2, and a core 4 embedded in the wire body 2.

The wire body 2 is made of a material (flexible material) which is deformed by an external force, and is not restored to its original configuration by itself, or a material (resilient or elastic material) which, after having been deformed, is restored to its original configuration, at the time of the use of the wire 1 for opening the stenosed region of a coronary artery (at the time of insertion into an artery). For example, the wire body 2 is made of a plastic material such as enamel, Teflon (trade name) or the like. Moreover, the wire body may have a coating layer such as hydrophilic polymer or the like, on the distal end portion of the wire body. Further, the wire body may have a coating layer such as tungsten-mixed polyurethane elastomer or the like, on a portion of the wire body except for the distal end portion thereof. Further, the wire body 2 is circular in transverse cross-section, and has a diameter such as 0.5 to 1.2 mm. The wire body 2 has a distal end which is so rounded as not to injure the coronary artery or the like.

Each of the projections 3 is formed substantially into a spherical shape. In the illustrated embodiment, three projections are provided. However, the number of the projections is optional. The outer diameter of each projection 3 is about 0.1 to 2.0 mm larger than the diameter of the wire body 2. The interval or spacing between the adjacent projections 3 is e.g. 0.5 to 5.5 mm. Each projection 3 has an entire length of 5 to 40 mm. The spacing between the projection 3 adjacent to the distal end of the wire body 2 and the distal end thereof is e.g. 10 to 50 mm, preferably, 10 to 20 mm.

The core 4 is made of a material (elastic material) which is restored to its original configuration after having been subjected to an external force and been deformed. For example, it is made of stainless steel, an alloy (nickel - titanium alloy or the like), a piano wire material, an amorphous metal, gold, a plastic material, or the like. The core 4 has the function of reinforcing the wire body 2, or the function of increasing the elasticity of the wire body 2. The core 4 does not reach the distal end of the wire body 2, but terminates at a location between the distal end and the projection 3. The core 4 has a diameter such as 0.1 to 1 mm.

Fig. 2 shows a wire 1A which has a plurality of projections 3A each of which is formed into a circular cylinder shape. In order to facilitate insertion of the wire into a coronary artery which is stenosed, a portion of the wire 1A between the distal end thereof and the projection 3A is bent through an angle α. In this case, a wire body 2A is made of a material (flexible material) which is deformed by on external force, and is not restored to its original configuration by itself, or a material (resilient or elastic material) which, after having been deformed, is restored to its original configuration, at the time of the use of the wire 1A for opening the stenosed region of a coronary artery. A core 4A is made of a material (elastic material) which, after having been subjected to an external force, is deformed, and thereafter restored to its original configuration, at the use of the wire 1A for opening the stenosed region of a coronary artery. Further, it is convenient if the core 4A is made of a material which, when an operator applies an external force larger than the elastic limit to the material, can plastically be deformed into an appropriate angle α (the angle α is an optional angle equal to or less than 90°), or can be deformed into an appropriate configuration. The reason is as follows. That is, in order to facilitate insertion of the wire through the stenosed region of a coronary artery, the operator can deform the portion of the distal end of the wire 1A for opening the stenosed region of the coronary artery, in accordance with parts of the stenosed region. The core 4A reaches a location adjacent to the distal end of the wire body 2A.

Fig. 3 illustrates a wire 1C which is arranged such that a spiral element 5 instead of the projections is provided in concentric relation to a core 4C, and a portion of the wire body 2C, which is within a range of the spiral element 5, is resected. The spiral element 5 is made of metal wire (the spiral element may be the core) having an affinity to the human body, a metal wire having high resistance to electricity, a metal wire having high or strong magnetism, or a plastic wire. The spiral element 5 has an outer diameter which may be larger than that of the wire body 2C, or may be smaller than the latter. A blank wire of the spiral element 5 has a diameter of 0.1 to 1 mm, and a pitch of 0.5 to 5 mm.

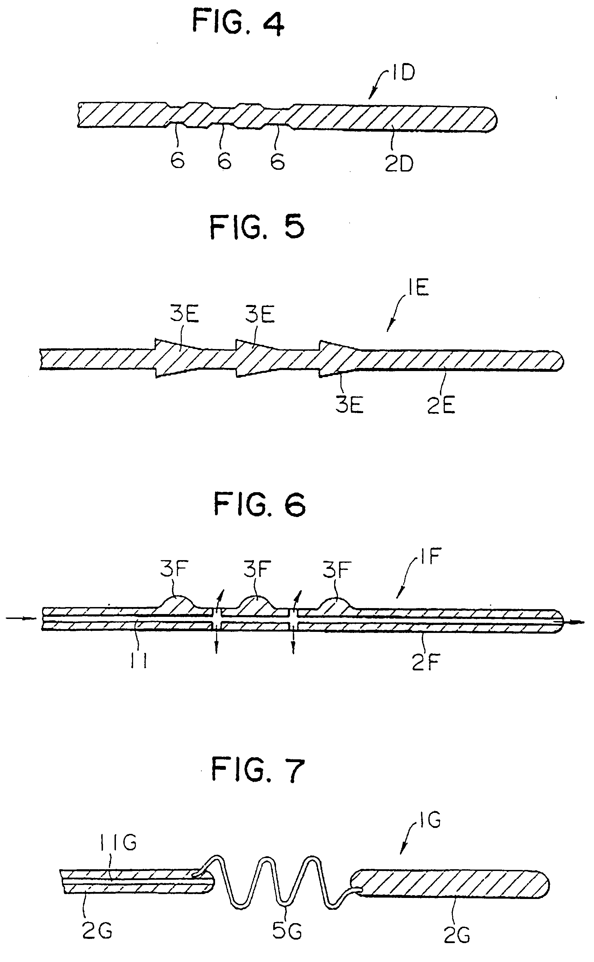

Fig. 4 illustrates a wire 1D which is arranged such that the wire body 2D is made of a resilient or elastic material, and the wire 1D is provided with a plurality of ring-like grooves 6 in substitution for the projections or projection.

Fig. 5 illustrates a wire 1E which is arranged such that each of a plurality of projections 3E is in the form of a circular truncated cone. Also in this case, the wire body 2E is made of a resilient or elastic material, similarly to the embodiment shown in Fig. 4.

Fig. 6 illustrates a wire 1F, a plurality of projections 3F being arranged only on one side of the wire body 2F, and a passage 11 through which a drug (a vascodilation drug, a thrombolytic drug or the like) is supplied, is formed in the wire body 2F. The passage 11 opens to the wire surface within the range of the projections 3F and at the distal end of the wire. The flow direction of the drug is indicated by arrows.

Fig. 7 illustrates a wire 1G which is provided with a spiral element 5G, similar to the embodiment shown in Fig. 4. However, the wire 1G is not provided with a core, but is formed with a passage 11G. The passage 11G opens to the surface of the wire body 2G at a location in short of the spiral element 5G. The arrangement can be such that a glass fiber is inserted through the passage, and a laser beam is capable of being applied from outside.

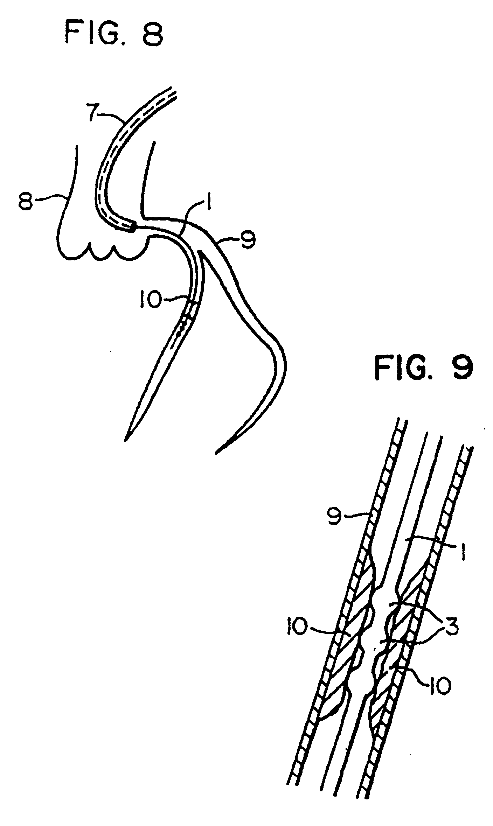

A method of opening an obstructive or stenosed region of a coronary artery, by the use of the brushing wire 1 for opening the coronary revascularization, will next be described with reference to Figs. 8 and 9. The revascularization is effected in the following order:
1) First, an artery (not shown) is pierced, and a sheath (not shown) is held within the artery.
2) A heart catheter 7 is inserted into the artery, and is led to the heart 8.
3) The distal end of the catheter 7 is inserted into a coronary artery 9, a contrast medium is injected, and the coronary artery is examined by fluoroscopy, to confirm a stenosed region 10.
4) The wire 1 for coronary revascularization is inserted into the catheter 7 and is put into the coronary artery 9 and is led to the coronary-artery stenosed region 10. At this time, since a portion of the wire between the distal end of the wire and the projection 3 serves as a guide, it is possible to easily insert the wire 1 into the stenosed region.
5) The wire 1 for coronary revascularization is operated to reciprocate the projections 3 several times between a location in front of the stenosed region 10 and in rear thereof, thereby enlarging the lumen while shaving the stenosed region. At this time, the arrangement may be such that high frequency current is applied to a high resistive material through the core, and a high frequency magnetic field is applied to a ferromagnetic body to cauterize and enlarge the stenosed region.
6) Lastly, the wire 1 for coronary revascularization is retracted into the catheter 7 and the catheter 7 is withdrawn from the artery.

As described above, the projections 3 on the wire 1 for coronary revascularization are located at the stenosed region and are reciprocated, whereby it is possible to cut and remove a thrombus, a hypertrophied vascular endothelium, cholesterol and the like existing in the stenosed region, to reopen blood stream to the heart or the like. Further, application of high frequency current through the core, and application of a high frequency magnetic field through the core enable the above-described thrombus and the like to be cauterized and removed.

The conventional balloon catheter is confined merely to enlarge such hypertrophied sections without removing the same. Accordingly, as time elapses, there is a very high probability that the lumen is depressed to cause restenosis. However, in the case where the wire 1 for coronary revascularization, according to the invention, is used, since the hypertrophied sections within the artery are removed, the probability that restenosis occurs becomes very low. Furthermore, by the use of wires of various sizes, it is possible to reopen a stenosed region of a coronary artery to enlarge the inner diameter of the coronary artery stepwise. Since the distal end of the wire is very rich in elasticity, a complication such as injury to a coronary artery, cardiogenic shock or the like is difficult to occur so that the wire exhibits effectiveness in any coronary arteries having a small diameter.

Moreover, a tendency of hemorrhage does not occur, and the percentage of success is very high, as compared with a conventional method in which a thrombolytic drug is used.

Further, since the elastic portion between the distal end of the wire and the projections 3 serves as a guide, it is possible to easily insert the wire into the stenosed region of a coronary artery. Furthermore, since slippage of the surface of the wire body 2 is superior, there is no case the inner wall of the coronary artery injured. Moreover, as described above, since it is sufficient only that the wire 1 is inserted into the coronary artery and is reciprocated, operating manipulation or handling is simple and easy. Further, since the structure or construction of the wire 1 is simple, the manufacturing cost therefor is low.

The wires 1, 1B or 1C do not necessarily require a core. In case where a core is provided, the core may reach the distal end of the wire body, or may reach a location slightly in short of the distal end of the wire body. Furthermore, also in case of the wires 1, 1A, 1C and 1D it is possible to provide a passage 11 or 11G. In this case, the arrangement may be such that the core and the passage are provided in the wire body in parallel relation to each other, and the passage larger than the core is formed so that the core is arranged within the passage. Moreover, the opening location of each of the passages 11 and 11G can be anywhere within the range of the distal end of the wire, and the number of the openings (outlets) is optional.

Further, it is possible to form the projections 3, 3A, 3E and 3F and the grooves 6 into any various configurations or shapes, and both the projections and the grooves can be provided. Furthermore, the number of the projections and the grooves is optional, and the arranging locations thereof can be anywhere if the arranging locations are within the range of the distal end of the wire.

As described above, the wire for coronary revascularization, according to the invention, has a very low probability that restenosis after operation occurs, and a complication such as a myocardial infarction, cardiogenic shock, coronary-artery injury or the like is difficult to occur. Moreover, the tendency of hemorrhage is low, and the percentage of success is very high. Further, there are the advantages that the manufacturing cost is low, and operating manipulation or handling is simple and easy.

## Claims

1. Device for coronary revascularization and in particular for opening or enlarging blood vessels like obstructed or stenosed regions in coronary arteries to enable much blood flow, comprising catheter (7) to be inserted into a coronary artery and a wire (1) within the catheter (7) having for enlarging and thereby opening the stenosed vascular region projections (3) or grooves (6) formed within the region of the distal end of the flexible or elastic wire body (2), the wire (1) having a guide portion at its distal end, said guide portion being circular in cross-section and having no projections and grooves,
**characterized in that**
the wire (1) has a plurality of spherical projections (3) or ring-like grooves (6), each projection (3) or groove (6) being isolated or separated from one another, and that the guide portion of the wire body (2) has a length of 10 to 50 mm from the distal end thereof.

2. Device for coronary revascularization and in particular for opening or enlarging blood vessels like obstructed or stenosed regions in coronary arteries to enable much blood flow, comprising a catheter (7) to be inserted into a coronary artery and a wire (1) within the catheter (7) having for enlarging and thereby opening the stenosed vascular region a spiral element (5) provided in spaced relation to the distal end of the flexible or elastic wire body (2), so as to form a guide portion at the distal end of the wire body, being circular in cross-section and having no spiral element
**characterized in that**
a portion of the wire body (2) within the range of the spiral element (5) is resected and that the guide portion of the wire body (2) has a length of 10 to 50 mm from the most distal end thereof.

3. Device according to claim 1, characterized in that each spherical projection (3) is made of a material selected from the group consisting of plastic material, high-electricity resistive material and high magnetic material.

4. Device according to claim 2, characterized in that the spiral element (5) is made of a material selected from the group consisting of high-electricity resistive material and high magnetic material.

5. Device according to anyone of claims 1 to 4, characterized in that an elastic core (4) is embedded in the wire body (2).

6. Device according to anyone of claims 1 to 5, characterized in that the wire body (2) is bent at a location between the distal end of the wire body and the spherical projections (3), the ring-like grooves (6) or the spiral element (5).

7. Device according to anyone of claims 1 to 4, characterized in that a passage (11) for supplying a drug is formed within the wire body (2) and open to the surface of the wire body within the range of the distal end of the wire body.

8. Device according to anyone of claims 1 to 6, characterized in that a passage (11) is formed within the wire body (2) through which a glass fiber is inserted, and that a laser is applicable through the glass fiber.

9. Device according to anyone of claims 2, or 4 to 8, characterized in that a core (4C) is provided within the flexible or elastic wire body (2), that the spiral element (5) made of a high-electricity resistive material and arranged in spaced relation to the distal end of the wire body is in connected relation to said core, and that high frequency current is applied to the spiral element so as to be capable of heating.

10. Device according to anyone of claims 1 to 9, characterized in that the distal end of the flexible or elastic wire body (2) is made of a high magnetic material and is capable of being heated by a high-frequency magnetic filed.

## Patentansprüche

1. Vorrichtung für eine koronare Revaskularisation und insbesondere für das Öffnen oder Aufweiten von Blutgefässen, wie zum Beispiel blockierten oder stenosierten Abschnitten von Koronararterien, um einen guten Blutfluß zu ermöglichen, enthaltend einen Katheter (7), welcher in die Koronararterie eingeschoben wird, und enthaltend einen Draht (1) innerhalb des Katheters (7), welcher für das Aufweiten und damit die Öffnung des stenosierten vaskulären Bereiches Vorspünge (3) oder Nuten (6) aufweist, welche im Bereich des distalen Endes des biegsamen oder elastischen Drahtkörpers (2) ausgebildet sind, wobei der Draht (1) an seinem distalen Ende einen Führungsabschnitt aufweist, welcher einen kreisförmigen Querschnitt aufweist und keine Vorsprünge oder Nuten besitzt,
**dadurch gekennzeichnet**, **daß**
der Draht (1) eine Vielzahl von kugelförmigen Vorsprüngen (3) oder ringförmigen Nuten (6) aufweist, und daß diese Vorsprünge oder Nuten im Abstand voneinander oder getrennt angeordnet sind, und daß der Führungsabschnitt des Drahtkörpers (2) eine Länge von 10 bis 50 mm gemessen von seinem distalen Ende hat.

2. Vorrichtung für die koronare Revaskularisation und insbesondere für das Öffnen oder Aufweiten von Blutgefässen, wie zum Beispiel blockierten oder stenosierten Abschnitten von Koronararterien, um einen guten Blutfluß zu ermöglichen, enthaltend einen Katheter (7), welcher in die Koronararterie eingeschoben wird, sowie einen im Inneren des Katheters (7) angeordneten Draht (1), welcher für das Aufweiten und damit die Öffnung des stenosierten vaskulären Abschnittes ein schraubenförmiges Element (5) aufweist, welches in einem gewissen Abstand von dem distalen Ende des biegsamen oder elastischen Drahtkörpers (2) so angeordnet ist, daß es einen Führungsabschnitt am distalen Ende dieses Drahtkörpers bildet, welcher einen kreisförmigen Querschnitt aufweist und kein schraubenförmiges Element besitzt,
**dadurch gekennzeichnet**, **daß**
ein Teil des Drahtkörpers (2) innerhalb des Bereiches des schraubenförmigen Elementes hinterschnitten ist und der Führungsabschnitt des Drahtkörpers (2) eine Länge von 10 bis 50 mm gemessen ab seinem distalen Ende hat.

3. Vorrichtung nach Anspruch 1,
**dadurch gekennzeichnet**, **daß**
jeder der kugelförmigen Vorsprünge (3) aus einem Material hergestellt ist, welches aus der Gruppe ausgewählt wird, die Kunststoffe, hochohmiges Material und hochmagnetisches Material umfaßt.

4. Vorrichtung nach Anspruch 2,
**dadurch gekennzeichnet**, **daß**
das schraubenförmige Element (5) aus einem Material hergestellt ist, welches aus der Gruppe ausgewählt wird, die hochohmiges Material und hochmagnetisches Material umfaßt.

5. Vorrichtung nach einem der Ansprüche 1 bis 4,
**dadurch gekennzeichnet**, **daß**
in dem Drahtkörper (2) ein elastischer Kern (4) eingebettet ist.

6. Vorrichtung nach einem der Ansprüche 1 bis 5,
**dadurch gekennzeichnet**, **daß**
der Drahtkörper (2) an einer Stelle zwischen dem distalen Ende des Drahtkörpers und den kugelförmigen Vorsprüngen (3), den ringförmigen Nuten (6) oder dem schraubenförmigen Element (5) umgebogen ist.

7. Vorrichtung nach einem der Ansprüche 1 bis 4,
**dadurch gekennzeichnet**, **daß**
ein Durchgang (11) für die Verabreichung eines Medikamentes innerhalb des Drahtkörpers (2) vorgesehen ist, welcher im Bereich des distalen Endes des Drahtkörpers an der Oberfläche dieses Drahtkörpers mündet.

8. Vorrichtung nach einem der Ansprüche 1 bis 6,
**dadurch gekennzeichnet**, **daß**
durch diesen in dem Drahtkörper (2) ausgebildeten Durchgang (11) eine Glasfaser eingeschoben wird und daß mit Hilfe dieser Glasfaser ein Laserstrahl beaufschlagt werden kann.

9. Vorrichtung nach einem der Ansprüche 2, bzw. 4 bis 8,
**dadurch gekennzeichnet**, **daß**
im Inneren des biegsamen oder elastischen Drahtkörpers (2) ein Kern (4C) vorgesehen ist, und daß das aus einem hochohmigen Material hergestellte schraubenförmige Element, welches im Abstand zu dem distalen Ende des Drahtkörpers angeordnet ist, mit dem Kern in Verbindung steht und dadurch, daß auf das schraubenförmige Element ein Hochfrequenzstrom beaufschlagt wird, so daß es erwärmt werden kann.

10. Vorrichtung nach einem der Ansprüche 1 bis 9,
**dadurch gekennzeichnet**, **daß**
das distale Ende des biegsamen oder elastischen Drahtkörpers (2) aus einem hochmagnetischen Material hergestellt wird und geeignet ist, mit Hilfe eines Hochfrequenzmagnetfeldes erwärmt zu werden.

## Revendications

1. Dispositif destiné à la revascularisation coronaire et, en particulier, à ouvrir ou élargir des vaisseaux sanguins tels que des régions obstruées ou sténosées dans des artères coronaires pour laisser passer un grand débit sanguin, comprenant un cathéter (7) destiné à être inséré dans une artère coronaire et un fil (1) logé dans le cathéter (7) et qui possède, pour élargir et par conséquent ouvrir la région vasculaire sténosée, des saillies (3) ou gorges (6) formées dans la région de l'extrémité distale du corps (2) du fil flexible ou élastique, le fil (1) ayant une partie de guidage à son extrémité distale, ladite partie de guidage étant circulaire en section transversale et ne possédant pas de saillies ni de gorges,
caractérisé en ce que
le fil (1) possède une pluralité de saillies sphériques (3) ou de gorges annulaires (6), les saillies (3) ou gorges (6) étant isolées ou séparées les unes des autres, et en ce que la partie de guidage du corps (2) du fil a une longueur de 10 à 50 mm à partir de son extrémité distale.

2. Dispositif destiné à la revascularisation coronaire et, en particulier, à ouvrir ou élargir des vaisseaux sanguins tels que des régions obstruées ou sténosées dans des artères coronaires pour laisser passer un grand débit sanguin, comprenant un cathéter (7) destiné à être inséré dans une artère coronaire et un fil (1) logé dans le cathéter (7) et qui possède, pour élargir et par conséquent ouvrir la région vasculaire sténosée, un élément hélicoïdal (5) prévu à distance de l'extrémité distale du corps (2) du fil flexible ou élastique, de manière à former à l'extrémité distale du corps du fil une partie de guidage, circulaire en section transversale et démunie d'élément hélicoïdal,
caractérisé en ce que
une partie du corps (2) du fil comprise dans la région de l'élément hélicoïdal (5) est enlevée et en ce que la partie de guidage du corps (2) du fil a une longueur de 10 à 50 mm à partir de son extrémité distale extrême.

3. Dispositif selon la revendication 1, caractérisé en ce que chaque saillie sphérique (3) est faite d'une matière choisie dans le groupe constitué par une matière plastique, une matière à haute résistivité électrique et une matière fortement magnétique.

4. Dispositif selon la revendication 2, caractérisé en ce que l'élément hélicoïdal (5) est fait d'une matière choisie dans le groupe constitué par une matière à haute résistivité électrique et une matière fortement magnétique.

5. Dispositif selon une quelconque des revendications 1 à 4, caractérisé en ce que l'âme élastique (4) est noyée dans le corps (2) du fil.

6. Dispositif selon une quelconque des revendications 1 à 5, caractérisé en ce que le corps (2) du fil est coudé en un emplacement entre l'extrémité distale du corps du fil et les saillies sphériques (3), les gorges annulaires (6) ou l'élément hélicoïdal (5).

7. Dispositif selon une quelconque des revendications 1 à 4, caractérisé en ce qu'un passage (11) servant à acheminer un médicament est formé dans le corps (2) du fil et débouche à la surface du corps du fil dans la région de l'extrémité distale du corps du fil.

8. Dispositif selon une quelconque des revendications 1 à 6, caractérisé en ce qu'il est prévu un passage (11) formé dans le corps (2) du fil et dans lequel est insérée une fibre de verre, et en ce qu'un rayon laser peut être appliqué à travers la fibre de verre.

9. Dispositif selon une quelconque des revendications 2 ou 4 à 8, caractérisé en ce qu'une âme (4C) est prévue dans le corps (2) du fil flexible ou élastique, en ce que l'élément hélicoïdal (5) fait d'une matière à haute résistivité électrique et agencé à distance de l'extrémité distale du corps du fil est relié à ladite âme, et en ce qu'un courant à haute fréquence est appliqué à l'élément hélicoïdal de manière à pouvoir le chauffer.

10. Dispositif selon une quelconque des revendications 1 à 9, caractérisé en ce que l'extrémité distale du corps (2) du fil flexible ou élastique est faite d'une matière fortement magnétique et peut être chauffée par un champ magnétique à haute fréquence.
